# EUROPEAN PATENT APPLICATION

(11) **EP 4 741 818 A1**
(43) Date of publication of application: **13.05.2026**
(21) Application number: 24839578.2
(22) Date of filing: 01.07.2024
(51) Int. Cl.: G01N 30/00, B01D 15/12, B01J 20/281, C07D 209/82, C07D 403/10, G01N 30/88

(54) **COMPOUND TREATMENT METHOD, METHOD FOR PRODUCING COMPOUND OR REACTION PRODUCT OF SAME, SYSTEM FOR TREATING COMPOUND, AND REPLACEMENT TREATMENT COLUMN**

(30) Priority: 07.07.2023 JP 2023112226
(71) Applicant: Flugelz Co., Ltd., Kyoto-shi, Kyoto 606-8501 (JP)
(72) Inventor: KANAMORI Kazuyoshi, Kyoto-shi, Kyoto 606-8501 (JP); MIYAMOTO Riichi, Kyoto-shi, Kyoto 606-8501 (JP); KAJI Hironori, Kyoto-shi, Kyoto 606-8501 (JP); TANAKA Hiroyuki, Kyoto-shi, Kyoto 606-8501 (JP); NAKAGAWA Hiromichi, Kyoto-shi, Kyoto 606-8501 (JP)
(74) Representative: Grünecker Patent- und Rechtsanwälte PartG mbB
(86) International application number: PCT/JP2024/023841
(87) International publication number: WO 2025/013686

(57) **Abstract**

Provided is a method of treating a compound, including treating the compound by passing a solution containing the compound as a solute through a treatment column. The treatment column includes a macroporous monolith in a path through which the solution passes. At least part of the solution passes through at least a partial section of the macroporous monolith in a supersaturated state with respect to the compound or a reaction product of the compound. The treatment method is suitable for the treatment of a compound having a low solubility.

## Description

### TECHNICAL FIELD

The present invention relates to a method of treating a compound, a method of producing a compound or a reaction product thereof, a system for treating a compound, and a treatment column for replacement.

### BACKGROUND ART

The separation and purification of a compound by liquid chromatography (hereinafter referred to as "LC") are each an indispensable technique in various fields typified by chemistry and pharmacy. The LC can achieve substance selectivity higher than that of each of the other separation and purification techniques, such as recrystallization and nanofiltration. A column packed with particles such as silica gel as a separation medium has heretofore been widely used as a treatment column in LC. As a treatment column different from the particle-packed column, in Patent Literature 1, there is a disclosure of a column including a macroporous monolith, which has through-holes each having a pore diameter of 500 nm or more and pores each having a pore diameter of from 5 nm to 100 nm, the pores being formed on the inner wall surfaces of the through-holes, and in which the through-holes and a skeleton have a three-dimensional network-like co-continuous structure.

### CITATION LIST

### Patent Literature

Patent Literature 1: JP 06-265534 A

### SUMMARY OF INVENTION

### Technical Problem

In the LC, while substance selectivity higher than that of each of the other separation and purification techniques can be achieved, the amount of solvent used increases. In addition, a compound serving as an organic electroluminescence (organic EL) material or a raw material for a pharmaceutical product often has a planar structure in which an aromatic ring is expanded by a π-conjugated system. A highly planar π-conjugated aromatic compound is liable to form an associated state based on so-called π-π stacking, and hence tends to have a low solubility in a solvent. To perform treatment based on the LC on a compound having a low solubility, a further large amount of a solvent is required. However, to cope with a demand for environmental protection, a reduction in the amount of a solvent to be used in the treatment of a compound has been desired.

An object of the present invention is to provide a method of treating a compound and a system for treating a compound that are suitable for the treatment of a compound having a low solubility.

### Solution to Problem

The inventors of the present invention have found that a solution present in a macropore of a macroporous monolith can take a stable state, in which the precipitation of its solute is suppressed, while being in a supersaturated state. Thus, the inventors have completed the present invention.

The present invention provides a method of treating a compound, including treating the compound by passing a solution containing the compound as a solute through a treatment column, wherein the treatment column includes a macroporous monolith in a path through which the solution passes, and wherein at least part of the solution passes through at least a partial section of the macroporous monolith in a supersaturated state with respect to the compound or a reaction product of the compound.

The present invention further provides a method of producing a compound or a reaction product thereof, including treating the compound by the treatment method of the present invention described above to provide the compound that has been treated or a reaction product of the compound.

The present invention further provides a system for treating a compound, including: a treatment unit including a treatment column through which a solution containing the compound as a solute is passed; and a supply unit configured to supply the solution to the treatment unit, wherein the treatment column includes a macroporous monolith in a path through which the solution passes, and wherein at least part of the solution passes through at least a partial section of the macroporous monolith in a supersaturated state with respect to the compound or a reaction product of the compound.

The present invention further provides a treatment column for replacement, including: a macroporous monolith; and a container configured to accommodate the macroporous monolith, wherein the treatment column includes the macroporous monolith in a path through which a solution containing a compound as a solute passes, and wherein the treatment column is used in at least one selected from the group consisting of: the method of treating a compound of the present invention described above; the method of producing a compound or a reaction product thereof of the present invention described above; and the system for treating a compound of the present invention described above.

### Advantageous Effects of Invention

The present invention can provide the method of treating a compound and the system for treating a compound that are suitable for the treatment of a compound having a low solubility.

### BRIEF DESCRIPTION OF DRAWINGS

FIG. 1A is a cross-sectional view for schematically illustrating an example of a treatment column that can be used in a method of treating a compound and a system for treating a compound of the present invention.
FIG. 1B is a cross-sectional view for schematically illustrating an example of the treatment column that can be used in the method of treating a compound and the system for treating a compound of the present invention.
FIG. 2 is a schematic view for illustrating an example of the system for treating a compound of the present invention.
FIG. 3 is a schematic view for illustrating an example of the system for treating a compound of the present invention.
FIG. 4 is a schematic view for illustrating an example of the system for treating a compound of the present invention.
FIG. 5 is a schematic view for illustrating an example of the system for treating a compound of the present invention.
FIG. 6 is a schematic view for illustrating an example of the system for treating a compound of the present invention.
FIG. 7 is an image obtained by observing a macroporous monolith produced in Examples with a scanning electron microscope.

### DESCRIPTION OF EMBODIMENTS

A method of treating a compound according to a first aspect of the present invention includes treating the compound by passing a solution containing the compound as a solute through a treatment column. The treatment column includes a macroporous monolith in a path through which the solution passes, and at least part of the solution passes through at least a partial section of the macroporous monolith in a supersaturated state with respect to the compound or a reaction product of the compound.

In a second aspect of the present invention, for example, in the treatment method according to the first aspect, at least part of the solution is recovered from the treatment column as a treated solution in a supersaturated state with respect to the compound or the reaction product.

In a third aspect of the present invention, for example, in the treatment method according to the first or second aspect, the solution is discharged from the treatment column while the compound or the reaction product is substantially prevented from being precipitated from the solution inside the macroporous monolith.

In a fourth aspect of the present invention, for example, the treatment method according to any one of the first to third aspects further includes performing solvent displacement on a stock solution, which contains the compound as a solute and is in a saturated or unsaturated state with respect to the compound, to provide the solution in a supersaturated state with respect to the compound. The solution in a supersaturated state thus obtained is supplied to the treatment column.

In a fifth aspect of the present invention, for example, in the treatment method according to the fourth aspect, the solution substantially free of a halogenated organic solvent is obtained by performing solvent displacement on the stock solution containing the halogenated organic solvent.

In a sixth aspect of the present invention, for example, in the treatment method according to any one of the first to fifth aspects, the solubility of the compound in a solvent of the solution at 25°C is 100 mg/mL or less.

In a seventh aspect of the present invention, for example, in the treatment method according to any one of the first to sixth aspects, the solution having a pressure of atmospheric pressure ± 0.3 atm is passed through the treatment column.

In an eighth aspect of the present invention, for example, in the treatment method according to any one of the first to seventh aspects, the compound is a liquid crystal compound or a π-conjugated aromatic compound.

In a ninth aspect of the present invention, for example, in the treatment method according to any one of the first to eighth aspects, the solubility of the compound in toluene or ethyl acetate at 25°C is 1/3 or less of the solubility thereof in a halogen-substituted methane at 25°C.

In a tenth aspect of the present invention, for example, in the treatment method according to any one of the first to ninth aspects, at least one kind of treatment selected from the group consisting of: purification; a reaction; and concentration is performed on the compound.

A method of producing a compound or a reaction product thereof according to an eleventh aspect of the present invention includes treating the compound by the method of treating a compound of any one of the first to tenth aspects to provide the compound that has been treated or a reaction product of the compound.

In a twelfth aspect of the present invention, for example, the production method according to the eleventh aspect further includes precipitating the treated compound or the reaction product without performing solvent displacement.

A system for treating a compound according to a thirteenth aspect of the present invention is a treatment system for treating a compound, including: a treatment unit including a treatment column through which a solution containing the compound as a solute is passed; and a supply unit configured to supply the solution to the treatment unit. The treatment column includes a macroporous monolith in a path through which the solution passes, and at least part of the solution passes through at least a partial section of the macroporous monolith in a supersaturated state with respect to the compound or a reaction product of the compound.

In a fourteenth aspect of the present invention, for example, in the treatment system according to the thirteenth aspect, the solution in a supersaturated state is supplied to the treatment column.

In a fifteenth aspect of the present invention, for example, in the treatment system according to the fourteenth aspect, the supply unit includes a solvent displacement unit configured to perform solvent displacement on a stock solution, which contains the compound as a solute and is in a saturated or unsaturated state with respect to the compound, to provide the solution in a supersaturated state.

In a sixteenth aspect of the present invention, for example, in the treatment system according to the fifteenth aspect, in the solvent displacement unit, the solution substantially free of a halogenated organic solvent is obtained from the stock solution containing the halogenated organic solvent.

In a seventeenth aspect of the present invention, for example, in the treatment system according to any one of the thirteenth to sixteenth aspects, the treatment column includes a container configured to accommodate the macroporous monolith, and the container is formed of a resin layer or a glass layer having a thickness of 5 mm or less, and is in contact with the macroporous monolith.

In an eighteenth aspect of the present invention, for example, the treatment system according to any one of the thirteenth to seventeenth aspects further includes a solid-liquid separation unit configured to obtain the compound treated by the treatment column as a solid from a treated solution discharged from the treatment unit.

In a nineteenth aspect of the present invention, for example, in the treatment system according to the eighteenth aspect, in the solid-liquid separation unit, the compound treated by the treatment column is precipitated from the treated solution without performing solvent displacement.

A treatment column according to a twentieth aspect of the present invention is a treatment column for replacement, including: a macroporous monolith; and a container configured to accommodate the macroporous monolith. The treatment column includes the macroporous monolith in a path through which a solution containing a compound as a solute passes, and the treatment column is used in at least one selected from the group consisting of: the method of treating a compound of any one of the first to tenth aspects; the method of producing a compound or a reaction product thereof of the eleventh or twelfth aspect; and the system for treating a compound of any one of the thirteenth to nineteenth aspects.

The present invention is described in detail below, but the present invention is not limited to the following embodiments, and may be optionally modified and carried out without departing from the gist of the present invention.

### [Method of treating Compound]

The method of treating a compound of this embodiment includes treating a compound (hereinafter referred to as "Compound A") by passing a solution containing Compound A as a solute (hereinafter referred to as "Solution B") through a treatment column. The treatment column includes a macroporous monolith in the path through which Solution B passes. At least part of Solution B passes through at least a partial section of the macroporous monolith in a supersaturated state with respect to Compound A or a reaction product of Compound A. When Solution B passes in a supersaturated state, the amount of Compound A to be treated per column weight (column loading) can be increased. When Solution B passes in a supersaturated state, the size of the treatment column required for treating the same weight of Compound A can be reduced. The reaction product of Compound A may be produced, for example, inside the macroporous monolith at the time of its passage through the treatment column, depending on the kind of treatment. The term "supersaturated state" as used herein means the following state: with regard to a solution at a certain temperature containing a solute and a solvent that dissolves the solute, the solute is dissolved in the solvent in an amount exceeding the solubility of the solute in the solvent at the temperature. When the solvent is a mixed solvent, whether or not Solution B is in a supersaturated state is judged on the basis of the solubility of its solute in the mixed solvent.

The term "macroporous monolith" as used herein means a monolith body in which macropores and a skeleton have a three-dimensional network-like co-continuous structure. The uniformity of the pore diameters of the macropores in the macroporous monolith is typically much higher than the uniformity of the diameters of voids between particles serving as a separation medium in a treatment column whose inside is packed with the particles. In addition, the skeleton of the macroporous monolith is generally excellent in strength because the skeleton has a three-dimensional network structure. Accordingly, fluctuations in pore diameters of the macropores at the time of the treatment of a compound are suppressed. The high uniformity of the pore diameters of the macropores of the macroporous monolith is presumed to contribute to the fact that the solution in a supersaturated state inside the macropores may be stable. In addition, there is a possibility that a capillary effect is involved in the stabilization of the solution in a supersaturated state. In the capillary effect, the growth of a compound to be precipitated into a microcrystal is suppressed by space restrictions. The term "macropore" as used herein means a pore having a pore diameter of 50 nm or more in accordance with a proposal by IUPAC. In addition, the term "mesopore", to be described later, means a pore intermediate in pore diameter between a macropore and a micropore (pore having a pore diameter of less than 2 nm), that is, having a pore diameter of 2 nm or more and less than 50 nm. The average pore diameter of the respective pores and the uniformity of the pore diameters thereof can be evaluated by such general pore size distribution measurement as described below, which is selected on the basis of the expected size of the average pore diameter: mercury intrusion porosimetry for macropores; or a nitrogen gas adsorption method for mesopores. More specifically, the uniformity of the pore diameters can be evaluated by the normal distribution analysis of a pore peak shown in a pore size distribution profile. The average pore diameter can be evaluated as a median diameter (d50).

The average pore diameter of the macropores is, for example, from 0.1 µm to 100 µm, and may be from 1 µm to 20 µm, or from 2 µm to 10 µm in terms of a median diameter (d50) determined by pore size distribution measurement based on mercury intrusion porosimetry.

The uniformity of the pore diameters of the macropores in the macroporous monolith is, for example, from 10% to 50%, and may be from 20% to 40% in terms of the ratio of the half-width of the pore diameter distribution thereof to the average pore diameter thereof determined by pore size distribution measurement based on mercury intrusion porosimetry. For example, when the average pore diameter is 5 µm, the macropores in the graph of their pore size distribution may be distributed in the pore diameter range of from 2.5 µm to 7.5 µm, or may be distributed in the range of from 3 µm to 7 µm, from 4 µm to 6 µm, or from 4.5 µm to 5.5 µm.

The macroporous monolith may have mesopores on the inner wall surfaces of the macropores. A macroporous monolith having mesopores typically has a hierarchical porous structure including macropores and mesopores. The hierarchical porous structure may contribute to an improvement in treatment performance of the treatment column. The average pore diameter of the mesopores is, for example, from 2 nm to 10 nm in terms of a median diameter (d50) determined by pore size distribution measurement based on a nitrogen gas adsorption method.

The specific surface area of the macroporous monolith is, for example, 100 m²/g or more, and may be 200 m²/g or more, 300 m²/g or more, 400 m²/g or more, 500 m²/g or more, 600 m²/g or more, 700 m²/g or more, or 800 m²/g or more.

The macroporous monolith may be produced by, for example, the progress of a sol-gel reaction used in combination with a phase separation process. In addition, according to the method, a macroporous monolith having a hierarchical porous structure can be produced by selecting conditions for the production. The above-mentioned method is disclosed in, for example, JP 2014-148456 A, WO 1999/038006 A1, JP 4874976 B2, JP 5288804 B2, US 7595350 B2, JP 6021738 B2, or US 7892516 B2. The macroporous monoliths disclosed in the respective publications may each be used.

The macroporous monolith may include an inorganic material, an organic material, or a hybrid material of the inorganic material and the organic material. Examples of the inorganic materials include silica and a metal oxide. The metal oxide is, for example, an oxide of at least one kind of metal selected from the group consisting of: aluminum; zirconium; titanium; hafnium; tin; gallium; germanium; and lead. Examples of the organic material include cellulose, an epoxy resin, an acrylic resin, and a styrenedivinylbenzene copolymer. The hybrid material is, for example, a composite material of a silicone resin and a silicon compound. The silicone resin may have a methylsiloxane skeleton. Examples of the silicon compounds include SiN and SiC. The skeleton of the silicone resin may have a functional group on its surface. Examples of the functional group include an alkyl group such as an ethyl group, a hydroxy group, an ether group, a keto group, an epoxy group, a carboxyl group, an acyl group, an amino group, an amide group, a halo group, and an aromatic group. However, the material for forming the macroporous monolith is not limited to the above-mentioned examples. A macroporous monolith including silica is suitable for regeneration with an acid, depending on its configuration.

The macroporous monolith may have a hydrophobic group on the surface of the skeleton. Examples of the hydrophobic group include an alkyl group and an aromatic group. The alkyl group may be a linear alkyl group. The number of the carbon atoms of the alkyl group may be 1 or more and 35 or less, or may be 4 or more and 25 or less, or 8 or more and 20 or less. A treatment column including a macroporous monolith having a hydrophobic group on the surface of its skeleton is suitable for use as a reversed-phase column.

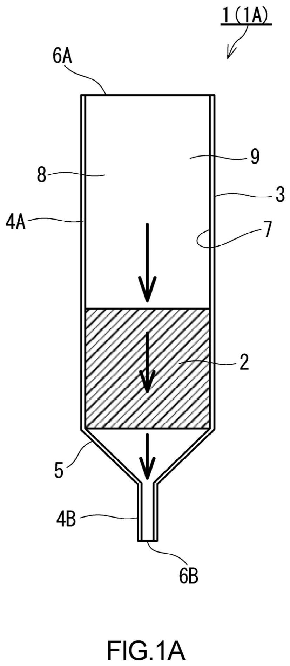

An example of the treatment column is illustrated in FIG. 1A. The treatment column 1 (1A) of FIG. 1A includes a container 3 and a macroporous monolith 2 accommodated in the container 3. The macroporous monolith 2 and the container 3 are in contact with each other. However, any other material and/or layer may be arranged between the macroporous monolith 2 and the container 3. The container 3 of FIG. 1A can function as a coating for protecting the macroporous monolith 2, and can also function as the exterior of the treatment column 1A.

The treatment column 1A includes the macroporous monolith in the path through which Solution B passes. The container 3 has a shape in which two cylinders 4A and 4B having different diameters are connected by a tapered part 5. The diameter of the cylinder 4A is larger than the diameter of the cylinder 4B. The thicknesses of the container 3 may be substantially the same throughout the cylinders 4A and 4B, and the tapered part 5. The phrase "the thicknesses of the container 3 are substantially the same" is meant to permit a molding error in a production process for the container 3, and for example, a difference between the maximum thickness and the minimum thickness may be 0.5 mm or less. An upper end 6A and a lower end 6B of the container 3 are opened. The opening diameter of the upper end 6A is larger than the opening diameter of the lower end 6B. The macroporous monolith 2 is columnar and is arranged inside the cylinder 4A so that no gap may be formed between the monolith and the inner circumferential surface 7 of the cylinder 4A. A space 9 is arranged above the macroporous monolith 2 inside the cylinder 4A. Solution B is supplied from the upper end 6A to the treatment column 1A, and is discharged from the lower end 6B after its passage through the macroporous monolith 2. Solution B thus supplied sequentially passes through the space 9, the macroporous monolith 2, the inside of the tapered part 5, and the inside of the cylinder 4B that form a path 8 (see the arrows of FIG. 1A). The space 9 may be provided with a function of temporarily storing Solution B that passes through the macroporous monolith 2. The volume of the space 9 may be larger than the volume of a portion in the treatment column 1A where the macroporous monolith 2 is accommodated.

The treatment column 1A of FIG. 1A does not include a filter and a particle layer at a position upstream of the path 8 with respect to the macroporous monolith 2. In other words, Solution B supplied to the treatment column 1A can directly flow into the macroporous monolith 2 without passing through any other member. This aspect is suitable for suppressing the precipitation of Compound A inside the treatment column 1A when Solution B in a supersaturated state with respect to Compound A is supplied. The filter and the particle layer may each have a surface that serves as a nucleus for the precipitation of Compound A. In addition, as illustrated in FIG. 1A, the treatment column 1A may not include a filter and a particle layer at a position downstream of the path 8 with respect to the macroporous monolith 2.

The shape of a transverse cross-section of the path 8 in the treatment column 1A is a circle. The shape of the transverse cross-section is not limited to a circle, and may be, for example, any one of polygons including a square, a pentagon, and a hexagon, or an ellipse. The term "transverse cross section" as used herein means a cross section perpendicular to the flow direction of Solution B.

The treatment column 1A is suitable for use in an open column mode in which Solution B is passed at atmospheric pressure or a pressure near atmospheric pressure. A pipe may be connected to the lower end 6B by known fastening means, such as a ferrule nut or a luer lock. The treatment column 1A may be used in a flash column mode in which Solution B is passed by medium pressure with respect to atmospheric pressure. In other words, the treatment column 1A may be an open column or a flash column. The term "open column mode" as used herein means a mode in which Solution B having a pressure of -0.3 atm or more and 0.3 atm or less (pressure of atmospheric pressure ± 0.3 atm) in terms of relative pressure based on atmospheric pressure is passed through the treatment column 1. The term "flash column mode" as used herein means a mode in which Solution B having a pressure of more than 0.3 atm and 20 atm or less, preferably 15 atm or less, more preferably 10 atm or less, still more preferably 5 atm or less, particularly preferably 3 atm or less in terms of relative pressure based on atmospheric pressure is passed through the treatment column 1. To pass Solution B having a positive relative pressure, for example, Solution B to be supplied to the treatment column 1 only needs to be pressurized. To pass Solution B having a negative relative pressure, for example, Solution B to be discharged from the treatment column 1 only needs to be sucked.

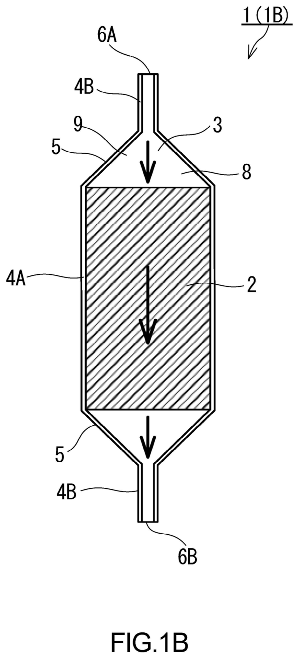

Another example of the treatment column is illustrated in FIG. 1B. The treatment column 1 (1B) of FIG. 1B is the same as the treatment column 1A of FIG. 1A except that the column 1B differs from the column 1A in configuration of the container 3. The container 3 of the treatment column 1B has a shape in which the cylinder 4B, the tapered part 5, the cylinder 4A, the tapered part 5, and the cylinder 4B are sequentially connected from the upper end 6A toward the lower end 6B. The treatment column 1B is suitable for use in a pressurization mode in which Solution B that has been pressurized is passed. A pipe may be connected to the upper end 6A by known fastening means, such as a ferrule nut or a luer lock.

The thickness of the container 3 is, for example, from 0.05 mm to 10 mm, and may be from 0.1 mm to 8 mm, or from 0.2 mm to 5 mm. The thickness of the container 3 may be 4 mm or less, 3 mm or less, 2 mm or less, 1.5 mm or less, 1.2 mm or less, 1 mm or less, 800 µm or less, 600 µm or less, 500 µm or less, 400 µm or less, 300 µm or less, 200 µm or less, or 150 µm or less. The thickness of the container 3 can be identified as a thickness in a portion where the macroporous monolith 2 is accommodated.

Examples of a material for the container 3 include a resin, glass, a metal, and a composite material thereof. The container 3 may include two or more kinds of materials. Various thermoplastic resins, thermosetting resins, elastomers, and the like may each be used as the resin. Examples of the thermoplastic resins include polyolefin resins, such as polyethylene and polypropylene. A resin having chemical resistance may be used in consideration of resistance to the solvent in Solution B. Examples of the resin having chemical resistance include: fluororesins, such as polytetrafluoroethylene (PTFE) and a perfluoroalkoxyalkane (PFA); and polyetheretherketone (PEEK). Examples of the glass include soda glass, borosilicate glass, and quartz glass. The container 3 may include a transparent or translucent portion, or may be entirely transparent or translucent. The container 3 including a transparent or translucent portion facilitates the recognition of the state of Solution B inside the treatment column 1, such as the presence or absence of the precipitation of Compound A, depending on its configuration. In the treatment column 1 to be used in an open column mode or a flash column mode, a resin layer or a glass layer having a thickness of 5 mm or less may be used in the container 3 because the pressure of Solution B to be supplied is low. A resin for forming the resin layer may be a polyolefin resin or a fluororesin. The glass layer may be a glass tube. The resin layer may be a resin tube, and the resin tube may be heat-shrinkable. According to a heat-shrinkable resin tube (heat-shrinkable tube), the treatment column 1 can be easily produced without the formation of any gap between the macroporous monolith 2 and the container 3 by arranging the macroporous monolith 2 in the tube and then heat-shrinking the tube. The thickness of the resin layer or the glass layer may be 3.5 mm or less, 3 mm or less, 2.5 mm or less, 2 mm or less, 1.5 mm or less, 1.2 mm or less, 1 mm or less, 800 µm or less, 600 µm or less, 500 µm or less, 400 µm or less, 300 µm or less, 200 µm or less, or 150 µm or less. The lower limit of the thickness is, for example, 50 µm or more, and may be 100 µm or more. In the treatment column 1 to be used in the flash column mode, the lower limit of the thickness is preferably 100 µm or more.

The container 3 may be a single layer or may have a multilayer structure including two or more layers. In the treatment column 1 to be used in an open column mode or a flash column mode, a single resin layer having a thickness of 5 mm or less may be used in the container 3.

The container 3 may accommodate the macroporous monolith 2 coated with a coating layer. Examples of the coating layer are the same as the examples of the resin layer described above. The strength of the container 3 accommodating the macroporous monolith 2 coated with the coating layer may be higher than that of the coating layer. Examples of the container 3 include a PEEK container, a fluororesin container, and a metal container.

The container 3 may form the exterior of the treatment column 1.

The length of the treatment column 1 is, for example, from 20 mm to 1000 mm, and may be from 50 mm to 500 mm. The length of the treatment column 1 may be determined in accordance with, for example, the length of the macroporous monolith 2, the length of the container 3, and the length of the tapered part 5. The length of the macroporous monolith 2 is, for example, from 5 mm to 500 mm, and may be from 10 mm to 250 mm. Although the tapered part 5 is easy to use when its length is equal to or less than the length of the macroporous monolith 2, the length is not limited thereto. The length of the container 3 is, for example, from 10 mm to 500 mm, and may be from 20 mm to 250 mm.

The inner diameter of the portion in the treatment column 1 where the macroporous monolith 2 is accommodated may be 10 mm or more, or may be 15 mm or more, 20 mm or more, 25 mm or more, 50 mm or more, or 100 mm or more. The upper limit of the inner diameter is, for example, 500 mm or less, and may be 400 mm or less, 300 mm or less, or 250 mm or less. When the shape of a transverse cross-section of the portion in the treatment column 1 where the macroporous monolith 2 is accommodated is not a circle, the length of the longest line segment out of virtual line segments each connecting two points set on the circumference of the transverse cross-section can be defined as the inner diameter.

The configuration of the treatment column 1 is not limited to the above-mentioned examples as long as the column includes the macroporous monolith 2 in the path 8 through which Solution B passes.

In the treatment method of this embodiment, at least part of Solution B passes through at least a partial section of the macroporous monolith 2 in a supersaturated state with respect to Compound A or a reaction product thereof. At least part of Solution B may pass through the entire section of the macroporous monolith 2 in a supersaturated state with respect to Compound A or the reaction product thereof. In addition, the entirety of Solution B to be passed through the treatment column 1 may pass through at least a partial section of the macroporous monolith 2 or the entire section thereof in a supersaturated state with respect to Compound A or the reaction product thereof.

In the treatment method of this embodiment, at least part of Solution B may be recovered from the treatment column 1 as a treated solution in a supersaturated state with respect to Compound A or the reaction product of Compound A.

When the treated solution in a supersaturated state recovered from the treatment column 1 loses its stability resulting from the macropores of the macroporous monolith 2, the solution can typically no longer maintain the supersaturated state, and hence the compound or reaction product therein precipitates. However, the precipitation does not necessarily occur quickly after the discharge of the solution from the treatment column 1. The precipitated compound or reaction product may be recovered by a solid-liquid separation approach such as filtration. In addition, the precipitation from the supersaturated state is similar to a recrystallization process except that no solvent displacement is performed, and hence the precipitation may contribute to the purification of Compound A. The treatment method of this embodiment may further include precipitating Compound A, which has been treated by the treatment column 1, or the reaction product thereof, from the treated solution discharged from the treatment column 1 without performing solvent displacement.

In the treatment method of this embodiment, Solution B may be discharged from the treatment column 1 while Compound A or the reaction product thereof is substantially prevented from being precipitated from Solution B inside the macroporous monolith 2. The phrase "substantially prevented from being precipitated" means that the amount of Compound A or the reaction product thereof to be precipitated is suppressed to, for example, 1 wt% or less, preferably 0.5 wt% or less, more preferably 0.1 wt% or less of the total amount of Compound A supplied to the treatment column 1.

Solution B in a supersaturated state with respect to Compound A may be supplied to the treatment column 1, or Solution B in a saturated or unsaturated state may be supplied.

In the treatment method of this embodiment, the following may be performed: Solution B in a saturated or unsaturated state with respect to Compound A is supplied to the treatment column 1; and at least part of Solution B is recovered from the treatment column 1 as a treated solution in a supersaturated state with respect to Compound A or the reaction product thereof. This aspect may be carried out by, for example, the concentration treatment of Compound A or the reaction product thereof by the treatment column 1. For the concentration, a reversed-phase column may be selected as the treatment column 1, and Solution B whose solvent is partially or entirely hydrophilic may be supplied to the treatment column 1. In the method, concentration based on a hydrophobic interaction between Compound A or the reaction product thereof and the reversed-phase column can be utilized. In accordance with the concentration of Solution B to be supplied and the state of the concentration, an eluent such as a hydrophilic solvent may be supplied to the treatment column 1 after the supply of Solution B. The solvent of Solution B and the eluent may be identical to or different from each other.

An example of the reversed-phase column is the treatment column 1 including the macroporous monolith 2 having a hydrophobic group on the surface of its skeleton. In an example of a case in which Compound A is DACT-II to be described later, the hydrophobic group may be a linear alkyl group having 8 to 18 carbon atoms, or may be an octadecyl group (18 carbon atoms).

The term "hydrophilic" as used herein means that the solubility of a solute in 100 g of water at 25°C is 1 g or more, preferably 10 g or more, more preferably 25 g or more. Examples of the hydrophilic solvent include water, a hydrophilic organic solvent, and a mixed solvent thereof. Examples of the hydrophilic organic solvents include: alcohols, such as methanol, ethanol, n-propanol, and 2-propanol; tetrahydrofuran (THF); acetone; and acetonitrile. However, the hydrophilic solvent and the hydrophilic organic solvent are not limited to the above-mentioned examples. In an example of a case in which Compound A is DACT-II, a THF/water mixed solution of DACT-II is supplied as Solution B to the treatment column 1, and then an acetone/methanol mixed solution is supplied as an eluent to the treatment column 1. With regard to the THF/water mixed solution of DACT-II, the concentration of DACT-II is, for example, 1 mg/mL, and a mixing ratio between THF and water is, for example, 1:1 (volume ratio). With regard to the acetone/methanol mixed solution, a mixing ratio between acetone and methanol is, for example, 1:1 (volume ratio). The concentration and the mixing ratios are not limited to the above-mentioned examples.

Solution B in a supersaturated state may be obtained by, for example, performing solvent displacement on a stock solution, which contains Compound A as a solute and is in a saturated or unsaturated state with respect to Compound A. In other words, the treatment method of this embodiment may further include performing solvent displacement on a stock solution, which contains Compound A as a solute and is in a saturated or unsaturated state with respect to Compound A, to provide Solution B in a supersaturated state with respect to Compound A. Solution B thus obtained can be supplied to the treatment column 1. Approaches, such as the addition and mixing of a solvent different from the solvent of the stock solution, heating, cooling, depressurization, pressurization, distillation, and dialysis, may each be selected and applied to the solvent displacement. A plurality of approaches may be combined.

In the solvent displacement from the stock solution, the solvent of the stock solution may be displaced with a solvent in which the solubility of Compound A at 25°C is 1/3 or less of that before the displacement, or the solvent may be displaced with a solvent in which the solubility is 1/4 or less, 1/5 or less, or 1/7 or less thereof.

Solution B substantially free of a halogenated organic solvent may be obtained by performing solvent displacement on a stock solution containing the halogenated organic solvent. Solution B substantially free of the halogenated organic solvent typically contains a non-halogenated organic solvent as a solvent. The phrase "substantially free of the halogenated organic solvent" means that the content of the halogenated organic solvent in the solution is, for example, 100 ppm or less, preferably 10 ppm or less, more preferably 1 ppm or less, particularly preferably 0.1 ppm or less. The unit "ppm" is based on weight.

Compound A, which is a π-conjugated aromatic compound, generally tends to have a solubility in a halogenated organic solvent higher than a solubility in a non-halogenated organic solvent. The tendency is particularly strong when Compound A has a hydrophilic group, such as a hydroxy group, a carboxyl group, an amino group, or an amide group. Accordingly, it has been common general technical knowledge that in conventional LC treatment for a π-conjugated aromatic compound, there is no choice but to use the halogenated organic solvent that can ensure a certain degree of solubility. In addition, depending on the kind of the LC treatment, an eluent needs to be continuously supplied after the supply of a solution containing a compound to be treated to the treatment column. It has been common general technical knowledge that to prevent the precipitation of the compound in the treatment column, there is no choice but to use the halogenated organic solvent as the eluent as well. However, the halogenated organic solvent is a solvent whose usage amount should be reduced because of its harmfulness and environmental impact, and hence, a demand for the reduction does not cease to increase. In the treatment method of this embodiment, a solution in a supersaturated state can be stably circulated through the treatment column 1. Accordingly, the treatment method of this embodiment is suitable even for treatment using a non-halogenated organic solvent as the solvent of Solution B, and as required, as the eluent. In other words, the method is also suitable for meeting the demand for dehalogenation.

The halogenated organic solvent is a solvent containing a halogen atom in a molecule thereof. Examples of the halogenated organic solvents include a halogen-substituted methane, a halogen-substituted ethane, and a halogenated benzene. Examples of the halogen-substituted methane include dichloromethane, chloroform, and carbon tetrachloride. The halogen-substituted ethane is, for example, chloroethane. The halogenated benzene is, for example, chlorobenzene. The halogenated organic solvent may be a homolog or derivative of any one of the above-mentioned examples. However, the halogenated organic solvent is not limited to the above-mentioned examples. The stock solution may contain two or more kinds of halogenated organic solvents.

The non-halogenated organic solvent is a solvent containing no halogen atom in a molecule thereof. Examples of the non-halogenated organic solvents include toluene, benzene, xylene, ethyl acetate, acetone, methyl ethyl ketone, diethyl ether, dimethyl sulfoxide, N,N'-dimethylformamide, tetrahydrofuran, 1,4-dioxane, hexane, cyclohexane, heptane, pentane, methanol, ethanol, 1-propanol, 2-propanol, acetonitrile, and benzonitrile. However, the non-halogenated organic solvent is not limited to the above-mentioned examples. Solution B may contain two or more kinds of non-halogenated organic solvents, irrespective of whether or not the solvent displacement is performed.

The solvent displacement may be performed at normal temperature (25°C).

The solvent displacement may be performed in one stage or may be performed in a plurality of stages formed of two or more stages. Multi-stage solvent displacement is more suitable depending on the kind of Compound A.

A porous membrane tube may be utilized in the solvent displacement. In an example of the solvent displacement utilizing the porous membrane tube, displacement with a solvent having a boiling point higher than that of the solvent before the displacement is performed. The displacement is performed, for example, as described below. A solution of Compound A containing a halogenated organic solvent such as a dichloromethane solution is supplied to the porous membrane tube. The solution to be supplied is typically in a saturated or sub-saturated state with respect to Compound A. Along with the foregoing, a non-halogenated organic solvent having a boiling point higher than that of the above-mentioned halogenated organic solvent, such as toluene or a mixed solvent of toluene and hexane, is supplied to the porous membrane tube, and the solution and the solvent are mixed in the tube. When an environment where the porous membrane tube to which the solution of Compound A and the non-halogenated organic solvent are supplied is placed is brought into a heated and depressurized atmosphere, the halogenated organic solvent having a relatively low boiling point is preferentially permeated through the side wall of the porous tube to be removed, and hence Solution B of Compound A containing the non-halogenated organic solvent as a main solvent is obtained. Solution B thus obtained may be brought into a supersaturated state with respect to Compound A because of a difference between the solubilities of Compound A in the halogenated organic solvent and the non-halogenated organic solvent. Solution B in a supersaturated state with respect to Compound A may be obtained after its discharge from the porous membrane tube, while a situation in which the solution inside the porous membrane tube is brought into a supersaturated state is avoided by controlling the temperature and pressure of the environment where the porous membrane tube is placed. Solution B thus obtained can be supplied to the treatment column 1. The porous membrane tube may be made of polytetrafluoroethylene. However, the porous membrane tube is not limited to the above-mentioned examples.

The solubility of Compound A in the solvent of Solution B at 25°C may be 100 mg/mL or less, or may be 50 mg/mL or less, 33 mg/mL or less, 20 mg/mL or less, 10 mg/mL or less, 5 mg/mL or less, or 3 mg/mL or less. The lower limit of the solubility is, for example, 0.1 mg/mL or more, and may be 1 mg/mL or more.

Compound A may be an organic compound, or may be an organic compound having at least one selected from the group consisting of: an aromatic ring; a sugar chain; and an amide bond. Compound A may also be a liquid crystal compound or a π-conjugated aromatic compound. Many liquid crystal compounds and π-conjugated aromatic compounds have low solubilities because the compounds each tend to be liable to form a π-π stacking structure in a solid state. The treatment method of this embodiment has a particularly great merit when Compound A having a low solubility in a solvent is treated.

Examples of Compound A include an organic EL material and a pharmaceutical raw material. In particular, the organic EL material is often a π-conjugated aromatic compound. Examples of the organic EL material are disclosed in JP 2020-205318 A, JP 7184785 B2, JP 4490896 B2, JP 6886972 B2, and US 11527728 B2. Specific examples of the organic EL material include 9-[4-(4,6-diphenyl-1,3,5-triazin-2-yl)phenyl]-N,N,N',N'-tetraphenyl-9H-carbazole-3,6-diamine (DACT-II), and anthracene, carbazole, benzoxazole, and derivatives thereof. Compound A may be a precursor of DACT-II. The structures of DACT-II and its precursor are represented below.

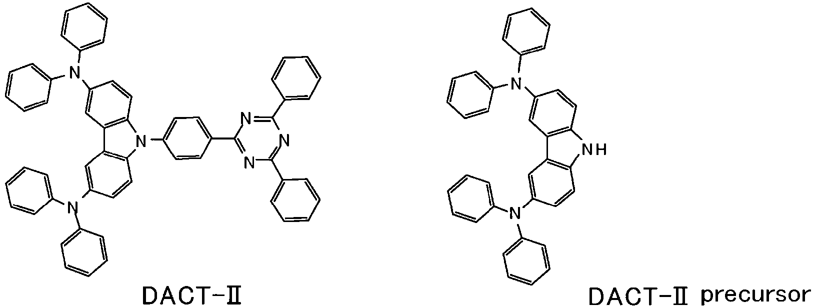

The solubility of Compound A in a non-halogenated organic solvent at 25°C may be 1/3 or less of the solubility thereof in a halogenated organic solvent at 25°C, or may be 1/4 or less, 1/5 or less, or 1/7 or less thereof. Examples of the non-halogenated organic solvent and the halogenated organic solvent are as described above. The non-halogenated organic solvent for which Compound A satisfies the above-mentioned ratios may be toluene, ethyl acetate, acetone, acetonitrile, methanol, diethyl ether, tetrahydrofuran, or water, or may be toluene or ethyl acetate. The halogenated organic solvent for which Compound A satisfies the above-mentioned ratios may be a halogen-substituted methane, may be dichloromethane or chloroform, or may be dichloromethane. As an example, the solubilities of DACT-II and its precursor are shown in Table 1 below.

**[Table 1]**

| Solubility (mg/mL) at 25°C | DACT-II | DACT-II precursor |
|---|---|---|
| Dichloromethane | 100 | 30 |
| Toluene | 30 | 10 |
| Ethyl acetate | 6 | 4 |

Compound A is not limited to the above-mentioned examples.

In the treatment method of this embodiment, Solution B having a pressure of atmospheric pressure ± 0.3 atm may be passed through the treatment column 1. In other words, the treatment method of this embodiment may be performed in an open column mode.

In the treatment method of this embodiment, Solution B having a pressure of more than 0.3 atm and 20 atm or less, preferably 15 atm or less, more preferably 10 atm or less, still more preferably 5 atm or less, particularly preferably 3 atm or less in terms of relative pressure based on atmospheric pressure may be passed through the treatment column 1. In other words, the treatment method of this embodiment may be performed in a flash column mode.

In the treatment method of this embodiment, at least one kind of treatment selected from the group consisting of: purification; a reaction; and concentration may be performed on Compound A. Conditions, such as the kind of the macroporous monolith, the solvent of Solution B, and a temperature, may be selected to perform each treatment. For example, a reduction reaction may be caused to proceed on Compound A by using such a macroporous monolith having a Si-H group on the surface of its skeleton as disclosed in JP 2014-148456 A.

The treatment method of this embodiment may include a step except for those described above, as long as Compound A can be treated.

The method of treating a compound of this embodiment may be performed by, for example, a system for treating a compound of this embodiment, as described later. However, a treatment apparatus and a treatment system that can each perform the method are not limited to examples to be described later.

### [Method of producing Compound or Reaction Product thereof]

The method of producing a compound or a reaction product thereof of this embodiment includes treating Compound A by the treatment method of this embodiment described above to provide Compound A that has been treated or a reaction product of Compound A. Specific aspects of the treatment are as described in the description of the treatment method of this embodiment.

The production method of this embodiment may further include precipitating Compound A that has been treated or the reaction product thereof without performing solvent displacement.

The production method of this embodiment may be used to provide, for example, an organic EL material or a pharmaceutical raw material. Examples of the organic EL material include DACT-II and a precursor thereof. However, the compound and reaction product that can each be produced by the production method of this embodiment are not limited to the above-mentioned examples.

The production method of this embodiment may be performed by, for example, a system for treating a compound of this embodiment to be described later. However, a treatment apparatus and a treatment system that can each perform the method are not limited to examples to be described later.

### [System for treating Compound]

A system for treating a compound that can perform the treatment method and/or the production method described above can be constructed by a treatment unit including the treatment column 1 and a supply unit that supplies Solution B to the treatment unit.

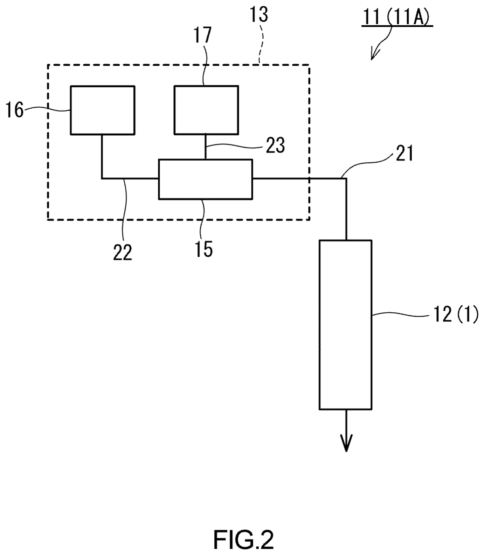

An example of the system for treating a compound of this embodiment is illustrated in FIG. 2. A treatment system 11 (11A) of FIG. 2 includes: a treatment unit 12 including the treatment column 1 through which Solution B is passed; and a supply unit 13 that supplies Solution B to the treatment unit 12. The supply unit 13 and the treatment unit 12 are connected by a pipe 21 through which Solution B is passed. The pipe 21 is connected to the upper end 6A of the treatment column 1 included in the treatment unit 12. The treatment column 1 includes the macroporous monolith 2 in the path through which Solution B passes. At least part of Solution B passes through at least a partial section of the macroporous monolith 2 in a supersaturated state with respect to Compound A or a reaction product of Compound A. In the treatment system 11, the method of treating a compound of this embodiment and/or the method of producing a compound or a reaction product thereof of this embodiment can be performed. Compound A is treated when Solution B passes through the treatment column 1 included in the treatment unit 12.

The treatment column 1 and a preferred aspect thereof are as described in the method of treating a compound. An example of the treatment column 1 includes the container 3 that accommodates the macroporous monolith 2, and the container 3 is formed of a resin layer or a glass layer having a thickness of 5 mm or less, and is in contact with the macroporous monolith 2. The treatment system 11 including the treatment column 1 is suitable for application to an open column mode or a flash column mode.

Solution B in a supersaturated state may be supplied to the treatment column 1.

The supply unit 13 of FIG. 2 includes: a solvent displacement unit 15 that performs solvent displacement on a stock solution, which contains Compound A as a solute and is in a saturated or unsaturated state with respect to Compound A, to provide Solution B in a supersaturated state; a stock solution storage unit 16 that supplies the stock solution to the solvent displacement unit 15; and a solvent storage unit 17 that supplies a solvent to be displaced to the solvent displacement unit 15. The stock solution storage unit 16 and the solvent storage unit 17 are connected to the solvent displacement unit 15 by pipes 22 and 23, respectively. In the treatment system 11 of FIG. 2, Solution B in a supersaturated state obtained by performing the solvent displacement on the stock solution can be supplied to the treatment column 1.

In the solvent displacement unit 15, Solution B in a supersaturated state, which is substantially free of a halogenated organic solvent, may be obtained from a stock solution containing the halogenated organic solvent. In the solvent displacement unit 15, various kinds of solvent displacement described in the description of the method of treating a compound of this embodiment may be performed.

The solvent displacement unit 15 may include at least one selected from the group consisting of a heating mechanism, a cooling mechanism, a pressurization mechanism, and a depressurization mechanism, for removing a solvent in the stock solution. However, the solvent displacement unit 15 is not limited to the above-mentioned examples, and may include any mechanism for solvent displacement.

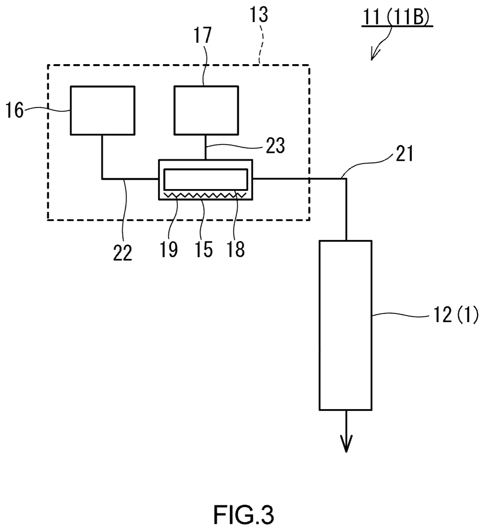

A more specific example of the treatment system 11 including the solvent displacement unit 15 is illustrated in FIG. 3. The solvent displacement unit 15 included in a treatment system 11 (11B) of FIG. 3 includes a porous membrane tube 18. The porous membrane tube 18 is connected to the pipes 22 and 23 so that the stock solution can be supplied from the stock solution storage unit 16 and the solvent to be displaced can be supplied from the solvent storage unit 17, respectively. The solvent displacement unit 15 further includes a heating mechanism 19 that raises the temperature of an environment where the porous membrane tube 18 is placed. In addition, a pressure adjustment mechanism (not shown) is connected to the solvent displacement unit 15, and hence can change the pressure of the environment where the porous membrane tube 18 is placed. The other configuration is the same as that of the treatment system 11A of FIG. 2. In the treatment system 11B, for example, the solvent displacement described above, as an example in which a porous membrane tube is utilized in solvent displacement, can be performed. The solvent of the stock solution to be supplied to the porous membrane tube 18 may be a halogenated organic solvent such as dichloromethane. The solvent to be displaced, which is supplied to the porous membrane tube 18, may be a non-halogenated organic solvent, such as toluene or a mixed solvent of toluene and hexane. Solution B of Compound A containing the non-halogenated organic solvent as a main solvent may be obtained by: mixing the stock solution and the solvent to be displaced in the porous membrane tube 18; and bringing the environment where the porous membrane tube 18 is placed into a heated and depressurized atmosphere to remove the halogenated organic solvent having a relatively low boiling point from the stock solution. Solution B thus obtained may be in a supersaturated state with respect to Compound A.

The supply unit 13 may have any configuration as long as Solution B can be supplied to the treatment unit 1. The supply unit 13 may not include the solvent displacement unit 15. The supply unit 13 may include a storage unit for Solution B.

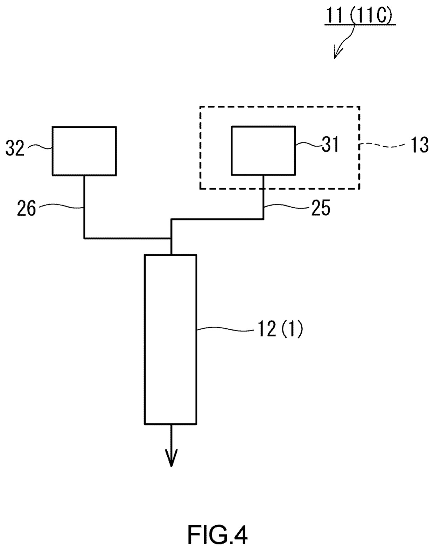

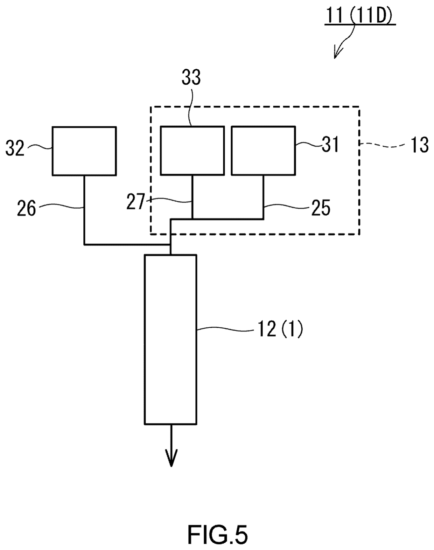

Solution B in a saturated or unsaturated state may be supplied to the treatment column 1. Examples of the treatment system 1 that supplies Solution B in a saturated or unsaturated state to the treatment column 1 are illustrated in FIG. 4 and FIG. 5.

A treatment system 11 (11C) of FIG. 4 includes: the treatment unit 12 including the treatment column 1 through which Solution B is passed; and the supply unit 13 that supplies Solution B to the treatment unit 12. The supply unit 13 and the treatment unit 12 are connected by a pipe 25 through which Solution B is passed. The pipe 25 is connected to the upper end 6A of the treatment column 1 included in the treatment unit 12. The supply unit 13 of FIG. 4 includes a storage unit 31 in which Solution B in a saturated or unsaturated state with respect to Compound A is stored. The treatment column 1 is the reversed-phase column described above. The treatment column 1 includes, for example, the macroporous monolith 2 having a hydrophobic group arranged on the surface of its skeleton. The solvent of Solution B stored in the storage unit 31 is hydrophilic. An example of the solvent when Compound A is DACT-II is a mixed solvent of a hydrophilic non-halogenated solvent and water, and an example of the non-halogenated solvent is THF. When Compound A is DACT-II, a mixing ratio between water and THF may be 1:1 (volume ratio). The solvent of Solution B stored in the storage unit 31 may be a mixed solvent of a hydrophilic solvent and a non-hydrophilic solvent. An example of the mixed solvent when Compound A is DACT-II is a mixed solvent of methanol and toluene, and a mixing ratio therebetween may be 2:1 (volume ratio).

When Solution B containing a hydrophilic solvent is supplied from the storage unit 31 to the treatment column 1, Compound A in Solution B is concentrated by a hydrophobic interaction caused by its contact with the macroporous monolith 2, and hence the concentration of Compound A can be increased. Thus, at least part of Solution B passes through at least a partial section of the macroporous monolith 2 in a supersaturated state with respect to Compound A or a reaction product of Compound A. To prevent the precipitation of Compound A due to the concentration of Solution B, an eluent may be supplied to the treatment column 1 after the supply of Solution B. The concentration of Solution B may mainly proceed in a portion where Solution B flows into the macroporous monolith 2. A hydrophilic solvent is typically used as the eluent. An eluent in which the solubility of Compound A is lower than that of Compound A in the solvent in Solution B may be used. An example of the eluent when Compound A is DACT-II is an acetone/methanol mixed solvent, and a mixing ratio therebetween may be 1:1 (volume ratio). Solution B may be passed through at least a partial section of the macroporous monolith 2 in a supersaturated state with respect to Compound A or the reaction product of Compound A, while the above-mentioned precipitation is prevented by supplying the eluent. To supply the eluent, the treatment system 11C includes an eluent storage unit 32 and a pipe 26 that supplies the eluent to the treatment column 1. The timing at which each of Solution B and the eluent is supplied, and the number of times of the supply are not limited. However, in ordinary cases, Solution B and the eluent are not supplied simultaneously.

A treatment system 11 (11D) of FIG. 5 is an example of a treatment system that supplies Solution B containing a mixed solvent of a first solvent and a second solvent to the treatment column 1 in a saturated or unsaturated state. The supply unit 13 of the treatment system 11D includes: the storage unit 31 in which Solution B containing the first solvent is stored; and a mixed solvent storage unit 33 in which the second solvent is stored. The mixed solvent storage unit 33 is connected to a pipe 27 that supplies Solution B to the treatment column 1. Solution B stored in the storage unit 31 is in an unsaturated state with respect to Compound A. When the first solvent and the second solvent are compared, the solubility of Compound A in the first solvent is higher than that in the second solvent. Accordingly, the concentration of Compound A to be stored in the storage unit 31 can be increased as compared to that in the case where Solution B containing the mixed solvent is stored from the beginning. The foregoing means that the amount of substance of Compound A to be stored can be increased when the volume of the storage unit 31 is kept constant. The other configuration of the treatment system 11D is the same as that of the treatment system 11C illustrated in FIG. 4.

In the treatment system 11D, Solution B from the storage unit 31 and the second solvent from the mixed solvent storage unit 33 are mixed in the pipe 25, and hence Solution B obtained by the mixing, which is in a saturated or unsaturated state with respect to Compound A, can be supplied to the treatment column 1. The mixing does not necessarily need to be performed in the pipe 25. The supply unit 13 of the treatment system 11D may further include a mixing unit that mixes Solution B from the storage unit 31 and the second solvent. When Solution B after the mixing is supplied to the treatment column 1, at least part of Solution B passes through at least a partial section of the macroporous monolith 2 in a supersaturated state with respect to Compound A or a reaction product of Compound A. In addition, after Solution B after the mixing has been supplied to the treatment column 1, an eluent may be supplied to the treatment column 1 as described above in the description of the treatment system 11C.

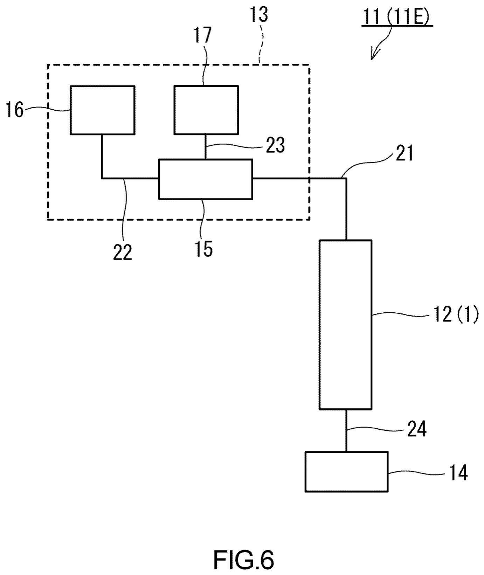

Another example of the system for treating a compound of this embodiment is illustrated in FIG. 6. A treatment system 11 (11E) of FIG. 6 has the same configuration as that of the treatment system 11A of FIG. 2 except that the system further includes: a solid-liquid separation unit 14 that obtains Compound A treated by the treatment column 1, in other words, Compound A or a reaction product of Compound A, as a solid from a treated solution discharged from the treatment unit 12; and a pipe 24 that connects the treatment unit 12 and the solid-liquid separation unit 14. The pipe 24 is connected to the lower end 6B of the treatment column 1 included in the treatment unit 12. In the solid-liquid separation unit 14, Compound A treated in the treatment column 1 may be precipitated without performing solvent displacement. Compound A after the treatment that has been precipitated may be taken out by solid-liquid separation means such as filtration, and the solid-liquid separation unit 14 may have a mechanism for this purpose. The treatment systems 11A to 11D of FIG. 2 to FIG. 5 may each include the solid-liquid separation unit 14.

In the system for treating a compound of this embodiment, Solution B may be supplied to the treatment unit 12 at a pressure of atmospheric pressure ± 0.3 atm. In other words, the treatment system of this embodiment may be an open column mode or flash column mode treatment system.

The system for treating a compound of this embodiment may include any apparatus or mechanism except those described above.

### [Treatment Column]

The treatment column of this embodiment is a treatment column for replacement, and includes: the macroporous monolith 2; and the container 3 that accommodates the macroporous monolith 2. The treatment column 1 includes the macroporous monolith 2 in the path through which Solution B passes. The macroporous monolith 2 is arranged in the path 8 through which Solution B passes, the path being arranged inside the container 3. Examples of the treatment column and a preferred aspect thereof are the same as the examples of the treatment column 1 described in the method of treating a compound. The treatment column of this embodiment may be a column for replacement to be used in the method of treating a compound of this embodiment, may be a column for replacement to be used in the method of producing a compound or a reaction product thereof of this embodiment, or may be a column for replacement to be used in the system for treating a compound of this embodiment. Examples of the treatment column of this embodiment are illustrated in FIG. 1A and FIG. 1B.

The treatment column of this embodiment may include a flow rate standard as a standard for replacement. In the flow rate standard, the column can be replaced by using a flow rate at a predetermined pressure as a standard for the degree of clogging of the treatment column. For example, the treatment column may be replaced at the following stage: a flow rate at the initial stage of its use when acetone is flowed at an increased pressure of 0.1 atm is 10 mL/min, but the flow rate reduces to 5 mL/min or less, which is a half or less of the initial flow rate, owing to its use.

### EXAMPLES

The present invention is described in more detail below by way of Examples. The present invention is not limited to the following Examples.

### (Preparation of Macroporous Monolith)

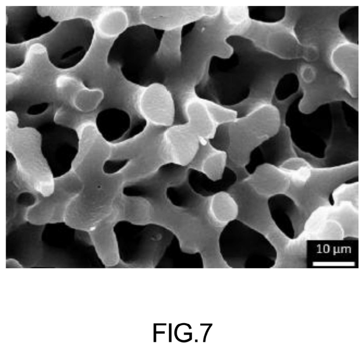

A macroporous monolith was prepared in accordance with a method described in each of K. Nakanishi, Y. Yamasaki, H. Kaji, and N. Soga, JSST, 1994, 2, 227-231, and R. Miyamoto, Y. Ando, C. Kurusu, H. Bai, K. Nakanishi, and M. Ippommatsu, J. Sep. Sci., 2013, 36, 1890-1896. Specifically, the following procedure was followed. Tetraethoxysilane was dissolved in a nitric acid solution containing polyethylene oxide (having a weight average molecular weight of 100000 or less) to cause a hydrolysis reaction and a condensation reaction to proceed. Thus, a uniform sol solution was obtained. Next, the resultant sol solution was gelled in a cylindrical container. Next, the resultant gel was hydrothermally treated in the presence of ammonia, and was then dried and calcined to provide a macroporous monolith having a columnar shape having an outer diameter of 22 mm and a height of 150 mm. An image obtained by observing the resultant macroporous monolith with a scanning electron microscope (SEM) is shown in FIG. 7. JSM-IT500HR manufactured by JEOL Ltd. was used as the SEM. As shown in FIG. 7, the prepared macroporous monolith had a co-continuous structure in which through-holes serving as macropores and a skeleton were intertwined and extended in a three-dimensional network pattern. In addition, pore size distribution measurement by mercury intrusion porosimetry was performed on the prepared macroporous monolith. The average pore diameter of the macropores determined on the basis of the result of the pore size distribution was 5 µm, and the uniformity of the pore diameters of the macropores was 20% in terms of the ratio of the half-width of the pore diameter distribution thereof to the average pore diameter. The range of the pore diameter distribution was from 4 µm to 6 µm.

### (Preparation of Treatment Column A including Macroporous Monolith)

The macroporous monolith prepared above was cut to a height of 100 mm (volume: 40 mL), and was coated with a heat-shrinkable tube made of tetrafluoroethylene-hexafluoropropylene copolymer (FEP) under a state in which a glass tube and a glass funnel were arranged at its upper end and lower end after the cutting, respectively. Thus, a treatment column A having a shape illustrated in FIG. 1A was prepared. The thickness of the heat-shrinkable tube after the coating was 0.5 mm. The glass tube and the glass funnel were coated with the heat-shrinkable tube integrally with the macroporous monolith so that solvent leakage did not occur.

### (Preparation of Treatment Column B packed with Silica Gel Particles)

Spherical silica gel particles (manufactured by FUJIFILM Wako Pure Chemical Corporation, Wakosil 300N, particle diameter: from 40 µm to 73 µm, pore diameter: 7 nm) were dispersed in a mixed solvent containing hexane and ethyl acetate at 8:1 (volume ratio) to prepare a slurry. Next, the prepared slurry was poured into a glass column tube with a glass cock (inner diameter: 20 mm) and allowed to settle to prepare a treatment column B of a comparative example including a columnar packing material having a diameter of 20 mm and a length of 100 mm.

### (Preparation of DACT-II)

In this Example, DACT-II was used as Compound A. DACT-II and a precursor thereof were synthesized by the following procedure with reference to H. Kaji et al., "Purely organic electroluminescent material realizing 100% conversion from electricity to light", Nat. Commun., 2015, 6, 8476.

9-Benzyl-3,6-dibromo-9H-carbazole and diphenylamine were caused to react with each other by being heated to reflux in dry toluene in the presence of tris(dibenzylideneacetone)dipalladium(0) and 2-dicyclohexylphosphino-2',4',6'-triisopropylbiphenyl catalysts, and sodium butoxide. Next, the reaction mixture was extracted with water and ethyl acetate, and the extract was purified and isolated by silica gel column chromatography to provide a reaction product. Next, the resultant reaction product was dissolved in dimethyl sulfoxide, and the solution was mixed with a tetrahydrofuran solution of potassium butoxide having a concentration of 1 mol/L, followed by oxygen gas bubbling under stirring. After the reaction, a crude product of a DACT-II precursor (N,N,N',N'-tetraphenyl-9H-carbazole-3,6-diamine) was obtained as a precipitate obtained by mixing with distilled water. Next, the above-mentioned precursor and 2-(4-bromophenyl)-4,6-diphenyl-1,3,5-triazine were caused to react with each other by being heated to reflux in dry toluene in the presence of tris(dibenzylideneacetone)dipalladium(0) and 2-dicyclohexylphosphino-2',4',6'-triisopropylbiphenyl catalysts, and sodium butoxide. Next, silica gel particles were dispersed in an ethyl acetate solution obtained by extracting the reaction mixture with water and ethyl acetate, followed by filtration. Thus, a crude product of DACT-II was obtained. The purity of the crude product of DACT-II was 83%. The purity of the crude product of DACT-II was evaluated by purity analysis based on high-performance liquid chromatography.

### (Preparation of Solution B1)

Solution B1 in a supersaturated state to be supplied to a treatment column was prepared as described below. 0.36 g of the crude composition of DACT-II was dissolved in 20 mL of dichloromethane. Next, 20 mL of toluene was added to the resultant solution, and then dichloromethane was removed with a rotary evaporator. The volume of the solution after the removal of dichloromethane was 15 mL. Next, while the whole was stirred, 20 mL of hexane was added dropwise until the lapse of 1 minute. Thus, Solution B1 in a supersaturated state with respect to DACT-II was obtained. The stability of the supersaturated state of the solution with respect to DACT-II is temporary. Accordingly, the loading of the solution into the treatment column A was subsequently performed in accordance with the procedure of Example 1 within a few minutes, before DACT-II started to precipitate.

### (Example 1)

The treatment column A prepared above was washed in advance with 50 mL of acetone, and was subsequently washed with 100 mL of hexane. Solution B1 thus prepared was loaded into the treatment column A by being poured into its glass tube before DACT-II precipitated. Next, the purification of DACT-II by the treatment column A was attempted by flowing a solvent obtained by mixing hexane and ethyl acetate at 8:1 (volume ratio) as an eluent from the upper end of the treatment column A at a flow rate of 20 mL/min. DACT-II was discharged as a solution in a supersaturated state from the glass funnel connected to the lower end of the treatment column A without precipitating inside the treatment column A. The solution discharged from the treatment column A was optionally recovered in fractions of 5 mL or 10 mL. DACT-II eluted at the time point when 80 mL of the solution was recovered from the start of the recovery of the first fraction, and subsequently, 80 mL of the DACT-II fractions in a supersaturated state were able to be recovered. It was confirmed from the recovered fractions that purification with a purity of 97% and a recovery rate of 80% in terms of values in the fractions was possible. The concentration of DACT-II in the recovered solution was 3.6 mg/mL, which was a concentration higher than the solubility of DACT-II in the used eluent, that is, 1 mg/mL, and it was confirmed that purification in a supersaturated state was possible. In addition, DACT-II started to precipitate from the solution in a supersaturated state about 5 minutes after recovery.

### (Comparative Example 1)

The purification of DACT-II was attempted in the same manner as in Example 1, except that the treatment column B was used instead of the treatment column A. However, DACT-II precipitated in the upper part of the treatment column B to clog the column, and hence it was impossible to purify DACT-II.

### (Example 2)

The macroporous monolith prepared above was crushed to provide an aggregate of monolith fragments (particle diameter: from 500 µm to 1000 µm). 0.72 g of the resultant aggregate was added to a dichloromethane solution (13 mL) of the crude composition of DACT-II (0.36 g). Next, 160 mL of hexane was added while the whole was stirred. As a result, the aggregate turned orange and maintained its fluidity in the solution. It was presumed that DACT-II was adsorbed inside the monolith fragments and in a stable state without precipitating on the surface of the aggregate.

Next, the above-mentioned solution was loaded together with the aggregate into the treatment column A by being poured into its glass tube. Next, the purification of DACT-II by the treatment column A was attempted by flowing a solvent obtained by mixing hexane and ethyl acetate at 8:1 (volume ratio) as an eluent from the upper end of the treatment column A at a flow rate of 20 mL/min. DACT-II was discharged as a solution in a supersaturated state from the glass funnel connected to the lower end of the treatment column A without precipitating inside the treatment column A. The solution discharged from the treatment column A was optionally recovered in fractions of 5 mL or 10 mL. DACT-II eluted at the time point when 55 mL of the solution was recovered from the start of the recovery of the first fraction, and subsequently, 60 mL of the DACT-II fractions in a supersaturated state were able to be recovered. It was confirmed from the recovered fractions that purification with a purity of 94% and a recovery rate of 87% in terms of values in the fractions was possible. The concentration of DACT-II in the recovered solution was 5.2 mg/mL, which was a concentration higher than the solubility of DACT-II in the used eluent, that is, 1 mg/mL, and it was confirmed that purification in a supersaturated state was possible. DACT-II started to precipitate from the solution in a supersaturated state about 5 minutes after recovery.

### (Comparative Example 2)

1.37 g of the silica gel particles used in the preparation of the treatment column B were added to a dichloromethane solution (13 mL) of the crude composition of DACT-II (0.36 g). Next, 90 mL of hexane was added while the whole was stirred. As a result, the silica gel turned strong yellow during the addition, lost its fluidity in the solution, and finally adhered to the bottom surface of the container. It was presumed that the addition of hexane precipitated DACT-II on the surfaces of the silica gel particles. Next, a certain degree of fluidity was imparted to the adhered aggregate by crushing the adhered aggregate with a spatula, and then the solution was loaded together with the aggregate into the treatment column B by being poured into its glass tube. Next, the purification of DACT-II by the treatment column B was attempted by flowing a solvent obtained by mixing hexane and ethyl acetate at 8:1 (volume ratio) as an eluent from the upper end of the treatment column B at a flow rate of about 20 mL/min. The yellow adhered component flowed into the treatment column B while being gradually dissolved. However, the component continued to accumulate at the inlet of the column even at the time point when 200 mL of the eluent was flowed.

### (Preparation of DACT-II Precursor Solution B2)

1.5 g of the crude product of the DACT-II precursor was dissolved in 60 mL of dichloromethane. Next, while the whole was stirred, 60 mL of hexane was added dropwise within 1 minute. Thus, Solution B2 in a supersaturated state with respect to the DACT-II precursor was obtained. The stability of the supersaturated state of the solution with respect to the DACT-II precursor is temporary. Accordingly, the loading of the solution into the treatment column A was subsequently performed in accordance with the procedure of Example 3 within a few minutes, before the DACT-II precursor started to precipitate.

### (Example 3)

Solution B2 thus prepared was loaded into the treatment column A by being poured into its glass tube before the DACT-II precursor precipitated. Next, the purification of the DACT-II precursor by the treatment column A was attempted by flowing a solvent obtained by mixing hexane and dichloromethane at 6:4 (volume ratio) as an eluent from the upper end of the treatment column A at a flow rate of 20 mL/min. The DACT-II precursor was discharged as a solution in a supersaturated state from the glass funnel connected to the lower end of the treatment column A without precipitating inside the treatment column A. The solution discharged from the treatment column A was optionally recovered in fractions of 5 mL or 10 mL. The DACT-II precursor eluted at the time point when 30 mL of the solution was recovered from the start of the recovery of the first fraction, and subsequently, 210 mL of the DACT-II precursor fractions in a supersaturated state were able to be recovered. It was confirmed from the recovered fractions that purification with a purity of 95% and a recovery rate of 94% in terms of values in the fractions was possible. The concentration of the DACT-II precursor in the recovered solution was 6.7 mg/mL, which was a concentration higher than the solubility of the DACT-II precursor in the used eluent, that is, 3 mg/mL, and it was confirmed that purification in a supersaturated state was possible.

### (Comparative Example 3)

The purification of the DACT-II precursor was attempted in the same manner as in Example 3, except that the treatment column B was used instead of the treatment column A. However, the DACT-II precursor precipitated in the upper part of the treatment column B to clog the column, and hence it was impossible to purify the DACT-II precursor.

### (Preparation of Treatment Column C packed with Silica Gel Particles)

Spherical silica gel particles (manufactured by FUJIFILM Wako Pure Chemical Corporation, Wakosil 300N, particle diameter: from 40 µm to 73 µm, pore diameter: 7 nm) were dispersed in a mixed solvent containing hexane and ethyl acetate at 8:1 (volume ratio) to prepare a slurry. Next, the prepared slurry was poured into a glass column tube with a glass cock (inner diameter: 50 mm) and allowed to settle to prepare a treatment column C of a comparative example including a columnar packing material (silica gel volume=300 mL) having a diameter of 50 mm and a length of 150 mm.

### (Comparative Example 4)

The purification of the DACT-II precursor was attempted in the same manner as in Example 3, except that the treatment column C was used instead of the treatment column A. The DACT-II precursor precipitated in the upper part of the treatment column C, but the precipitated DACT-II was gradually redissolved by continuing to flow the eluent as it was. The DACT-II precursor eluted at the time point when 520 mL of the solution was recovered from the start of the recovery of the first fraction, and subsequently, a total of 480 mL of DACT-II precursor fractions were able to be recovered. It was confirmed from the recovered fractions that purification with a purity of 95% and a recovery rate of 99% in terms of values in the fractions was possible. The concentration of DACT-II in the recovered solution was 3.1 mg/mL, which was within the range of error as compared to the solubility of DACT-II in the used eluent, that is, 3 mg/mL. Accordingly, it was recognized that purification in a supersaturated state was not achieved. The amount of silica gel particles (300 mL) required for the purification in Comparative Example 4 was 7.5 times as large as that of the macroporous monolith (40 mL) in Example 3. In addition, the amount of the solvent required for the purification was 1000 mL (520+480), which was nearly 4 times as large as 240 mL (30+210) in Example 3.

### (Preparation of Treatment Column D including Macroporous Monolith)

The macroporous monolith prepared above was cut to a height of 130 mm and loaded into an empty polypropylene reservoir (measuring 22 mm in inner diameter by 130 mm in length, thickness: 1.5 mm) including Luer lock connectors at its inlet and outlet to prepare a treatment column D.

### (Preparation of Treatment Column E packed with Silica Gel Particles)

Silica gel particles each having a particle diameter of from 20 µm to 40 µm were packed into an empty polypropylene reservoir (measuring 12 mm in inner diameter by 80 mm in length) including Luer lock connectors at its inlet and outlet to prepare a treatment column E of a comparative example.

### (Preparation of Solution B3)

Solution B3 in a supersaturated state to be supplied to a treatment column was prepared as described below. 0.36 g of the crude product of DACT-II was dissolved in 10 mL of chloroform. Next, 4 mL of toluene was added to the resultant solution, and then chloroform was removed with a rotary evaporator. The volume of the solution after the removal of chloroform was 4 mL. Next, while the whole was stirred, 8 mL of hexane was added dropwise until the lapse of 30 seconds. Thus, Solution B3 in a supersaturated state with respect to DACT-II was obtained. The stability of the supersaturated state of the solution with respect to DACT-II is temporary. Accordingly, the loading of the solution into the treatment column D was subsequently performed in accordance with the procedure of Example 4 within a few minutes, before DACT-II started to precipitate.

### (Example 4)

The treatment column D prepared above was washed in advance with 50 mL of acetone, and was subsequently washed with 100 mL of hexane. Solution B3 thus prepared was loaded directly into the column before DACT-II precipitated. A syringe was used in the loading. Next, the purification of DACT-II by the treatment column D was attempted by flowing a solvent obtained by mixing hexane and ethyl acetate at 8:1 (volume ratio) as an eluent from the inlet of the column at a flow rate of 20 mL/min with an HPLC pump. DACT-II was discharged as a solution in a supersaturated state from the outlet of the treatment column D without precipitating inside the treatment column D. DACT-II eluted at the time point when 70 mL of the solution was recovered from the start of the recovery of the first fraction, and subsequently, 40 mL of DACT-II fractions in a supersaturated state were able to be recovered. It was confirmed from the recovered fractions that purification with a purity of 92% and a recovery rate of 80% in terms of values in the fractions was possible. The concentration of DACT-II in the recovered solution was 7.5 mg/mL, which was a concentration higher than the solubility of DACT-II in the used eluent, that is, 1.1 mg/mL, and it was confirmed that purification in a supersaturated state was possible.

### (Comparative Example 5)

The treatment column E prepared above was washed in advance with 10 mL of acetone, and was subsequently washed with 20 mL of hexane. Solution B3 thus prepared was loaded directly into the column before DACT-II precipitated. A syringe was used for loading. Next, the purification of DACT-II by the treatment column E was attempted by flowing a solvent obtained by mixing hexane and ethyl acetate at 8:1 (volume ratio) as an eluent from the inlet of the column at a flow rate of 5 mL/min with an HPLC pump. Although the clogging of the treatment column E did not occur, the solution was discharged from the outlet of the treatment column E while DACT-II precipitated throughout the treatment column E. The amount of DACT-II precipitated inside the treatment column E reached 20 wt% of the loaded amount thereof, and hence, the purification thereof was impossible.

### (Examples 5 to 7)

The purification of DACT-II by the treatment column D was attempted in the same manner as in Example 4, except that the mixed solvents shown in Table 2 below were each used as an eluent. In each of Examples, DACT-II was discharged as a solution in a supersaturated state from the outlet of the treatment column D without precipitating inside the treatment column D. Table 3 below shows the solubility of DACT-II in each of the used eluents (25°C), the amount of DACT-II fractions in a supersaturated state that were able to be recovered, the concentration of DACT-II in the fractions, and the purity and recovery rate of DACT-II serving as a precipitate that spontaneously precipitated from the discharged fractions together with the results of Example 4.

**[Table 2]**

| Example | Eluent |
|---|---|
| 4 | Hexane/ethyl acetate (volume ratio: 8:1) |
| 5 | Hexane/toluene (volume ratio: 2:1) |
| 6 | Hexane/THF (volume ratio: 8:1) |
| 7 | Hexane/acetone (volume ratio: 8:1) |

**[Table 3]**

| Example | Solubility (mg/mL) | Amount of fractions that were able to be recovered (mL) | Concentration of DACT-II in fractions (mg/mL) | Precipitate | |
|---|---|---|---|---|---|
| | | | | Purity (%) | Recovery Rate (%) |
| 4 | <1.1 | 40 | 7.5 | 96.1 | 62 |
| 5 | <5.9 | 30 | 10 | 99.9 | 39 |
| 6 | <2.1 | 80 | 3.7 | 99.2 | 50 |
| 7 | <0.3 | 30 | 10 | - | 46 |

As shown in Table 3, it was recognized that high-purity purification of DACT-II from a supersaturated state was possible in each of Examples including Example 7 in which the eluent, in which the solubility of DACT-II was as extremely low as 0.3 mg/mL, was used. The purity of DACT-II in Solution B3 used in the purification was 83%.

### (Reference Example)

The recrystallization purification of DACT-II by a solvent diffusion method was performed by layering 100 mL of hexane on 10 mL of a toluene solution (concentration: 35 mg/mL) of DACT-II that had been heated and dissolved in a hot water bath at 40°C, and then allowing the laminate to stand still at room temperature for 3 days. However, the purity of DACT-II thus obtained was 93.9%.

### (Preparation of Treatment Column F including Macroporous Monolith)

A treatment column F was prepared in the same manner as in the treatment column A, except that the macroporous monolith was changed to a macroporous monolith having a diameter of 33 mm and a height of 100 mm.

### (Preparation of Solution B4)

Solution B4 in a supersaturated state to be supplied to the treatment column F was prepared as described below. 1.5 g of DACT-II was dissolved in 20 mL of dichloromethane. Next, 20 mL of toluene was added to the resultant solution, and then dichloromethane was removed with a rotary evaporator. The volume of the solution after the removal of dichloromethane was 20 mL. Next, while the whole was stirred, 20 mL of hexane was added dropwise until the lapse of 30 seconds. Thus, Solution B4 in a supersaturated state with respect to DACT-II was obtained. The stability of the supersaturated state of the solution with respect to DACT-II is temporary. Accordingly, the loading of the solution into the treatment column F was subsequently performed in accordance with the procedure of Example 8 within a few minutes, before DACT-II started to precipitate.

### (Example 8)

The treatment column F prepared above was washed in advance with 100 mL of acetone, and was subsequently washed with 200 mL of hexane. Solution B4 thus prepared was loaded directly into the column before DACT-II precipitated. Next, the purification of DACT-II by the treatment column F was attempted by flowing a solvent obtained by mixing hexane and toluene at 3:2 (volume ratio) as an eluent from the inlet of the column at a flow rate of 20 mL/min with a hand pump. DACT-II was discharged as a solution in a supersaturated state from the outlet of the treatment column F without precipitating inside the treatment column F. 119 mL of a DACT-II fraction in a supersaturated state was able to be recovered. The purity and recovery rate of DACT-II in the recovered fraction were 99.4% and 96.8%, respectively. The purity and recovery rate of DACT-II serving as a precipitate that spontaneously precipitated from the discharged fraction were 99.9% or more and 61%, respectively. In addition, the concentration of DACT-II in the recovered solution was 11 mg/mL, which was a concentration higher than the solubility of DACT-II in the used eluent, that is, 5 mg/mL, and it was confirmed that purification in a supersaturated state was possible.

### Industrial Applicability

The method of treating a compound of the present invention may be utilized in, for example, the purification, concentration, or reaction of a compound. Examples of the compound include an organic EL material and a pharmaceutical raw material.

## Claims

1. A method of treating a compound, comprising treating the compound by passing a solution containing the compound as a solute through a treatment column,
wherein the treatment column includes a macroporous monolith in a path through which the solution passes, and
wherein at least part of the solution passes through at least a partial section of the macroporous monolith in a supersaturated state with respect to the compound or a reaction product of the compound.

2. The method of treating a compound according to claim 1, wherein at least part of the solution is recovered from the treatment column as a treated solution in a supersaturated state with respect to the compound or the reaction product.

3. The method of treating a compound according to claim 1, wherein the solution is discharged from the treatment column while the compound or the reaction product is substantially prevented from being precipitated from the solution inside the macroporous monolith.

4. The method of treating a compound according to claim 1, further comprising performing solvent displacement on a stock solution, which contains the compound as a solute and is in a saturated or unsaturated state with respect to the compound, to provide the solution in a supersaturated state with respect to the compound,
wherein the solution in a supersaturated state thus obtained is supplied to the treatment column.

5. The method of treating a compound according to claim 4, wherein the solution substantially free of a halogenated organic solvent is obtained by performing solvent displacement on the stock solution containing the halogenated organic solvent.

6. The method of treating a compound according to claim 1, wherein a solubility of the compound in a solvent of the solution at 25°C is 100 mg/mL or less.

7. The method of treating a compound according to claim 1, wherein the solution having a pressure of atmospheric pressure ± 0.3 atm is passed through the treatment column.

8. The method of treating a compound according to claim 1, wherein the compound is a liquid crystal compound or a π-conjugated aromatic compound.

9. The method of treating a compound according to claim 1, wherein a solubility of the compound in toluene or ethyl acetate at 25°C is 1/3 or less of a solubility thereof in a halogen-substituted methane at 25°C.

10. The method of treating a compound according to claim 1, wherein at least one kind of treatment selected from the group consisting of: purification; a reaction; and concentration is performed on the compound.

11. A method of producing a compound or a reaction product thereof, comprising treating the compound by the method of treating a compound of claim 1 to provide the compound that has been treated or a reaction product of the compound.

12. The method of producing a compound or a reaction product thereof according to claim 11, further comprising precipitating the treated compound or the reaction product without performing solvent displacement.

13. A system for treating a compound, comprising:
a treatment unit including a treatment column through which a solution containing the compound as a solute is passed; and
a supply unit configured to supply the solution to the treatment unit,
wherein the treatment column includes a macroporous monolith in a path through which the solution passes, and
wherein at least part of the solution passes through at least a partial section of the macroporous monolith in a supersaturated state with respect to the compound or a reaction product of the compound.

14. The system for treating a compound according to claim 13, wherein the solution in a supersaturated state is supplied to the treatment column.

15. The system for treating a compound according to claim 14, wherein the supply unit includes a solvent displacement unit configured to perform solvent displacement on a stock solution, which contains the compound as a solute and is in a saturated or unsaturated state with respect to the compound, to provide the solution in a supersaturated state.

16. The system for treating a compound according to claim 15, wherein in the solvent displacement unit, the solution substantially free of a halogenated organic solvent is obtained from the stock solution containing the halogenated organic solvent.

17. The system for treating a compound according to claim 13,
wherein the treatment column includes a container configured to accommodate the macroporous monolith, and
wherein the container is formed of a resin layer or a glass layer having a thickness of 5 mm or less, and is in contact with the macroporous monolith.

18. The system for treating a compound according to claim 13, wherein the system for treating a compound further comprises a solid-liquid separation unit configured to obtain the compound treated by the treatment column as a solid from a treated solution discharged from the treatment unit.

19. The system for treating a compound according to claim 18, wherein in the solid-liquid separation unit, the compound treated by the treatment column is precipitated from the treated solution without performing solvent displacement.

20. A treatment column for replacement, comprising:
a macroporous monolith; and
a container configured to accommodate the macroporous monolith,
wherein the treatment column includes the macroporous monolith in a path through which a solution containing a compound as a solute passes, and
wherein the treatment column is used in at least one selected from the group consisting of: the method of treating a compound of any one of claims 1 to 10; the method of producing a compound or a reaction product thereof of claim 11 or 12; and the system for treating a compound of any one of claims 13 to 19.
